Europäisches Patentamt

European Patent Office

Office européen des brevets

(11) Veröffentlichungsnummer: **0 680 973 A1**

## (12) EUROPÄISCHE PATENTANMELDUNG

(21) Anmeldenummer: **95105382.6**

(22) Anmeldetag: **10.04.95**

(51) Int. Cl.6: **C07K 5/10**, C07K 5/08, C07K 14/16, A61K 38/04

(30) Priorität: **08.04.94 DE 4412174**

(43) Veröffentlichungstag der Anmeldung: **08.11.95 Patentblatt 95/45**

(84) Benannte Vertragsstaaten: **DE ES FR GB IT**

(71) Anmelder: **SCHRAMM, Wolfgang**
**Medizinische Kliniken Innenstadt der Universität München**
**Ziemssenstr. 1**
**D-80336 München (DE)**
Anmelder: **Schramm, Hans J.**
**Max-Planck-Institut für Biochemie,**
**Am Klopferspitz 18a**
**D-82153 Martinsried (DE)**

(72) Erfinder: **SCHRAMM, Wolfgang**
**Medizinische Kliniken Innenstadt der Universität München**
**Ziemssenstr. 1**
**D-80336 München (DE)**
Erfinder: **Schramm, Hans J.**
**Max-Planck-Institut für Biochemie,**
**Am Klopferspitz 18a**
**D-82153 Martinsried (DE)**

(74) Vertreter: **Müller-Boré & Partner**
**Patentanwälte**
**Grafinger Strasse 2**
**D-81671 München (DE)**

(54) **Peptide, Verfahren zu ihrer Herstellung und ihre Verwendung.**

(57) Die Erfindung betrifft ein Peptid, das eine Aminosäuresequenz der allgemeinen Formel (1) umfaßt

$Y-A^2-A^3-A^4-X$ (1),

in der
Y        Wasserstoff, eine Aminosäure oder ein weiteres Peptid ist,
$A^2$       Arg, Tyr oder Phe ist,
$A^3$       Glu, Gln, Asn oder Asp ist,
$A^4$       Leu, Phe, Met, Tyr, Trp, Ile oder Nle ist und
X        OH, $NH_2$, eine Aminosäure oder ein weiteres Peptid ist.
Ferner betrifft die Erfindung ein Verfahren zur Herstellung eines solchen Peptids und seine Verwendung

Die Erfindung betrifft Peptide, Verfahren zu ihrer Herstellung und ihre Verwendung.

Es ist bekannt, Proteasen von HI-Viren durch verschiedene Verbindungen zu hemmen. Eine Klasse derartiger Verbindungen kann die Aktivität der Proteasen durch Beeinflußung des aktiven Zentrums hemmen. Sie besitzen den Nachteil, daß sie auch körpereigene Aspartatatproteasen hemmen. Eine andere Klasse derartiger Verbindungen kann die dimere Struktur von HIV-Proteasen beeinflussen und somit eine Hemmung der Proteasen bewirken, da nur das Dimere aktiv ist. Letztere Verbindungen sind beispielsweise Peptide. Diese Peptide sind jedoch nur in hohen Konzentrationen wirksam, d.h. ihre Hemmaktivität ist gering.

Der vorliegenden Erfindung liegt somit die Aufgabe zugrunde, ein Mittel zur Hemmung von HIV-Proteasen bereitzustellen, das nicht die vorstehenden Nachteile aufweist.

Erfindungsgemäß wird dies durch ein Peptid mit einer Aminosäuresequenz der allgemeinen Formel (1) erreicht

$$Y\text{-}A^2\text{-}A^3\text{-}A^4\text{-}X \qquad (1),$$

in der

| | |
|---|---|
| Y | Wasserstoff, eine Aminosäure oder ein weiteres Peptid ist, |
| $A^2$ | Arg, Tyr oder Phe ist, |
| $A^3$ | Glu, Gln, Asn oder Asp ist, |
| $A^4$ | Leu, Phe, Met, Tyr, Trp, Ile oder Nle ist und |
| X | OH, $NH_2$, eine Aminosäure oder ein weiteres Peptid ist. |

Bei allen in dieser Patentanmeldung erwähnten Peptiden wird gemäß der üblichen Schreibweise das N-terminale Ende des Peptids links aufgeführt. Des weiteren werden die üblichen Abkürzungen für die Aminosäuren verwendet. Die Aminosäuren können in den in dieser Anmeldung erwähnten Peptiden in der D- und/oder L-Form vorliegen. Die Abkürzung HomoSer steht für die Aminosäure Homoserin. Die Abkürzung Nle steht für die Aminosäure Norleucin. Die erfindungsgemäßen Peptide können sowohl linear als auch cyclisch sein. Cyclische Peptide sind in üblicher Weise verknüpft, beispielsweise über eine Disulfid-Brücke. In den Peptidsequenzen verhindert ein geringer Anteil an nicht-optimalen Aminosäuren die Hemmwirkung nicht.

In bevorzugter Ausführungsform des Peptids ist Y eine Aminosäure, ausgewählt aus Ser, Thr, Homo-Ser, Asn, Glu, Typ, Phe, Leu oder Ile. Diese sind in üblicher Weise an das N-terminale Ende des Peptids gebunden.

In einer weiteren bevorzugten Ausführungsform des erfindungsgemäßen Peptids ist Y ein weiteres Peptid der allgemeinen Formel (2)

$$R^3\text{-}A^1 \qquad (2),$$

in der

| | |
|---|---|
| $R^3$ | Ile, Leu, Met, Phe, Tyr, Val, Orn, Lys oder Arg ist und |
| $A^1$ | Ser, Thr, HomoSer, Asn, Glu, Typ, Phe, Leu oder Ile ist. |

$A^1$ ist mit dem N-terminalen Ende des Peptids verbunden.

Erfindungsgemäß wird auch ein Peptid bereitgestellt, in dem Y ein weiteres Peptid der allgemeinen Formel (3) ist

$$R^2\text{-}R^3\text{-}A^1 \qquad (3),$$

in der

| | |
|---|---|
| $R^2$ | Lys, Thr, His, Met, Ile oder Arg ist und |
| $R^3$ und $A^1$ | wie vorstehend definiert sind. |

In einer weiteren bevorzugten Ausführungsform des Peptids ist Y ein weiteres Peptid der allgemeinen Formel (4)

$$R^1\text{-}R^2\text{-}R^3\text{-}A^1 \qquad (4),$$

in der

| | |
|---|---|
| $R^1$ | Arg, Asp oder Asn ist und |
| $R^2$, $R^3$ und $A^1$ | wie vorstehend definiert sind. |

In bevorzugter Ausführungsform des Peptids ist X eine Aminosäure, ausgewählt aus Glu, Gln, Asp oder Asn. Diese sind in üblicher Weise an das C-terminale Ende des Peptids gebunden.

In einer bevorzugten Ausführungsform ist X ein weiteres Peptid der allgemeinen Formel (5)

$$R^4 - R^5 \qquad (5),$$

in der

R$^4$     Glu, Gln, Asp oder Asn ist und

R$^5$     Met, Leu, Phe, Tyr, Leu, oder Met ist.

R$^4$ ist an das C-terminale Ende des Peptids gebunden.

In bevorzugter Ausführungsform des Peptids wird an das N-terminale Ende des Peptids über einen Linker ein Peptidfragment oder modifiziertes Peptidfragment z.B. einer HIV-Protease gebunden.

Der Ausdruck "Linker" umfaßt Aminosäuresequenzen jeglicher Art aus mindestens 3 bis 25 Aminosäuren. Vorzugsweise handelt es sich bei diesen Aminosäuren um hydrophobe Aminosäuren, beispielsweise Leu, Phe, Gly. Des weiteren kann Thr im Linker verwendet werden. Weiterhin werden auch nicht-natürliche Aminosäuren, wie beispielsweise 6-Aminohexansäure (X) und β-Alanin (β) eingesetzt. Beispiele derartiger Linker sind der Tabelle 2 zu entnehmen. In dieser Tabelle ist der Linker nicht unterstrichen. Auch Dicarbonsäuren, Diimidsäureester und/oder Diamine sind als Linker verwendbar. Dann kann eines der beiden durch den Linker verbundenen Peptide in der retroinverso-Form vorliegen.

Der Ausdruck "Peptidfragment des N-terminalen Endes einer HIV-Protease" umfaßt 3 bis 8 Aminosäuren einer HIV-Protease, wobei die Aminosäuren vom N-terminalen Ende gezählt werden. Beispiel eines derartigen Peptidfragments ist die Aminosäuresequenz Pro Gln Ile Thr Leu Trp Gln Arg.

Der Ausdruck "modifiziertes Peptidfragment einer HIV-Protease" umfaßt ein Peptidfragment einer HIV-Protease, in dem mindestens eine Aminosäure ausgetauscht ist. Beispielsweise kann aus der Sequenz Pro Gln Ile Thr Leu Trp Gln Arg die Aminosäure Pro durch Leu, His, Met, Phe oder Ile ersetzt sein; das erste Gln durch Glu oder Orn; Ile durch Phe; Thr durch Ser; Leu durch Tyr, Lys oder Phe; Trp durch Ser, Glu, Phe, Tyr oder Gly; das zweite Gln durch Glu, Asn oder Asp.

In bevorzugter Ausführungsform des Peptids ist an das Peptid oder das Peptidfragment bzw. modifizierte Peptidfragment einer HIV-Protease eine Verbindung, wie eine Amidin-Gruppe, eine Acyl-Gruppe, die von einer Carbonsäure aus 1 bis 20 Kohlenstoffatomen, vorzugsweise von Essigsäure, Isobuttersäure oder Palmitinsäure, abgeleitet ist, ein aliphatischer Rest mit 1 bis 20 Kohlenstoffatomen, ein aromatischer Rest mit 1 bis 20 Kohlenstoffatomen, ein Rest mit 1 bis 20 Kohlenstoffatomen aus einem aromatischen Teil und einem aliphatischen Teil, wobei entweder der aromatische oder der aliphatische Teil an das Peptid gebunden ist, ein Vitamin oder -derivat, beispielsweise Biotin, ein Steroid, beispielsweise Cholesterin, ein Hormon, ein Antigen, ein an einen Rezeptor bindendes Peptid, beispielsweise $\alpha_2$-Macroglobulin, ein $\alpha_2$-Macroglobulinkomplex, modifiziertes $\alpha_2$-Macroglobulin oder ein weiteres Mitglied der $\alpha$-Macroglobuline, ein Toxin, ein Inhibitor, ein Cytokin, beispielsweise ein Interleukin oder ein Interferon, und/oder ein Borkomplex für Neutronentherapie gebunden. Solche Verbindungen sind dem Fachmann bekannt. Die Verbindungen können auch über einen vorstehend definierten Linker an das Peptid gebunden sein. Die vorstehenden aliphatischen und aromatischen Reste können z.B. über eine Amidinogruppe an das Peptid gebunden sein.

Die erfindungsgemäßen Peptide gehen eine Wechselwirkung mit HIV-Proteasen ein, wodurch die Hemmung der Proteasen bewirkt wird. Sind die vorstehend bezeichneten Verbindungen direkt oder über einen Linker an das erfindungsgemäße Peptid gebunden, so werden diese ebenfalls an die Protease herangeführt. Sie können dann zur Verstärkung des Hemmeffekts beitragen und/oder ihre Funktionen erfüllen. Darüberhinaus kann z.B. an Biotin oder an ein Antigen eine dazu komplementäre Komponente in üblicher Weise gebunden werden, die zur Hemmung der Protease beiträgt. Die Komponente kann auch den Zelleintritt vermitteln oder andere Wirkungen haben.

Durch die Acyl-Gruppen, insbesondere durch den Palmitoylrest wird die Hemmaktivität und die Zellpermeablität erhöht.

Es wird verstanden, daß an die Peptide ein oder mehrere der vorstehend genannten Verbindungen gebunden sein können. Dabei können diese Verbindungen gleich oder verschieden sein.

In bevorzugter Ausführungsform des Peptids ist mindestens eine der Aminosäuren chemisch modifiziert. Dabei bedeutet der Ausdruck "chemisch modifiziert jegliche Art der chemischen Veränderung einer Aminosäure. Beispielsweise sind aliphatische oder aromatische Wasserstoffatome durch chemische Moleküle, wie NO$_2$, Halogen, OH, ersetzt. Weiterhin tragen die Peptide auch reaktive Gruppen, beispielsweise Diazoketone, Nitrile, -SS-Brücken, Halogenacylgruppen, Imidsäureester.

Beispiele erfindungsgemäßer Peptide sind in den Tabellen 1 bis 5 angegeben.

Erfindungsgemäß wird auch ein Verfahren zur Herstellung von vorstehenden Peptiden bereitgestellt. Hierzu werden Aminosäuren in üblicher Weise, beispielsweise nach der "Merrifield-Synthese" (siehe Römpp, Lexikon der Chemie, Stuttgart, New York, Band 4, 1991), miteinander verknüpft. Vorstehend beschriebene Verbindungen, wie Acyl-Gruppe, aliphatischer Rest, aromatischer Rest, gemischt aliphatisch-aromatischer Rest, Vitamin, Steroid, Hormon, Antigen usw. (vgl. vorstehend), können ebenfalls in üblicher Weise direkt oder über einen Linker mit den vorstehenden Peptiden verknüpft werden.

Erfindungsgemäße Peptide weisen in ihrer Peptidhauptkette eine beta-Faltblattstruktur auf. Verbindungen, die in ihrer Peptidhauptkette eine beta-Faltblattstruktur nachahmen (sog. beta-Faltblattstruktur-Mimetika) und gleichzeitig die gleichen Aminosäurereste wie die erfindungsgemäßen Peptide aufweisen, hemmen ebenso eine retrovirale Protease. Solche Mimetika fallen daher auch unter die vorliegende Erfindung.

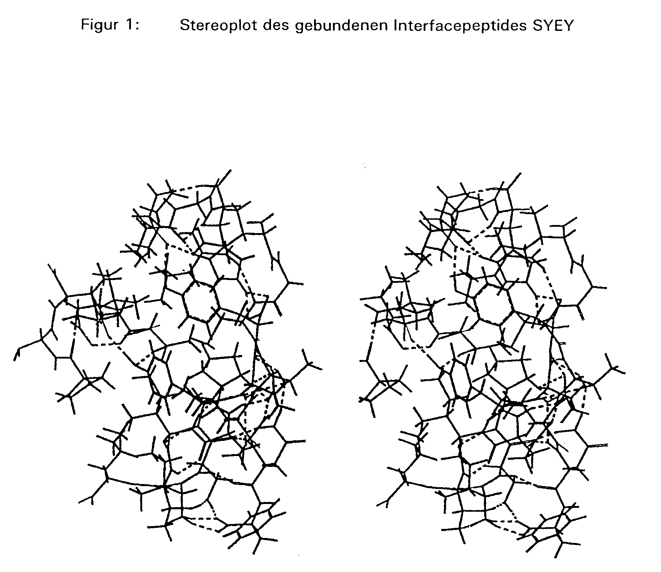

Unter die Erfindung fallen auch nichtspezifische Verbindungen, die eine Struktur besitzen, die der pharmakologisch wirksamen Struktur der erfindungsgemäßen Peptiden entspricht oder ihr sehr ähnelt (sog. Pharmakophor), dergestalt, daß charakteristische Gruppen der nichtpeptidischen Verbindungen (z.B. -OH, -CO, -COO$^-$, $C_6H_5$, aliphatische Reste, -NH-, -NH$_3$$^+$)räumlich in der selben oder sehr ähnlichen Anordnung und Lage wie bei den Peptiden sind. Dabei ist es nicht nötig, daß alle die Struktur bestimmende Gruppen identisch sin. Auch das beta-Strukturgerüst des Peptids ist dann nicht essentiell. Die pharmakophore Struktur der erfindungsgemäßen Peptide geht aus der Figur 1 hervor. Bei Veränderungen der Seitenketten im Rahmen der aufgeführten Variationen von $A^2$, $A^3$ und $A^4$ etc. (vgl. Figur 1), verändert sich auch der Pharmakophor in gewissen Grenzen. Das Gerüst der Strukturen geht aus Figur 1 iVm Tabelle 6 hervor.

Die erfindungsgemäßen Peptide zeichnen sich durch eine Hemmung von retroviralen Proteasen, beispielsweise von HIV-Proteasen oder HTLV-Proteasen, aus. Die erfindungsgemäßen Peptide weisen dabei eine hohe Hemmaktivität auf. Diese Peptide sind nicht toxisch. Sie besitzen somit einen günstigen therapeutischen Index. Die erfindungsgemäßen Peptide eignen sich somit zur Verwendung als Arzneimittel. Derartige Arzneimittel können weiterhin übliche Mittel zur Zubereitung von Arzneimitteln enthalten. Des weiteren können diese Arzneimittel in Kombination mit anderen Wirkstoffen, beispielsweise anderen zur HIV-Therapie verwendeten Wirkstoffen, wie AZT und/oder "active-site"-Inhibitoren, als sog. "Cocktails" gegeben werden. Die erfindungsgemäßen Peptide zeigen Synergismus zu den auf das aktive Zentrum der Protease gerichteten Inhibitoren. Derartige Arzneimittel können oral, intravenös, intramuskulär und in anderen üblichen Arten verabreicht werden.

Es wird auch verstanden, daß ein erfindungsgemäßes Peptid von den Zellen selbst produziert wird, die von einem Retrovirus, z.B. HI- oder HTL-Virus, befallen sind. Die Zellen werden hierzu mit einem Expressionsvektor transfiziert, der die DNA für das Peptid in einer Expressionseinheit enthält.

Kurze Beschreibung der Zeichnung:

Fig. 1:   zeigt einen Stereoplot des gebundenen 'Interface' Peptides SYEY.

Die Abbildung zeigt die für die Bindung wichtigen Seiten- und Endgruppen. Die nicht-bindenden Reste der monomeren Protease sind weggelassen. Die Entfernungen sind: C des terminalen $CO_2$ zu C-OH des Tyr$_{99}$ 0,51 nm; C des terminalen $CO_2$ zu C-OH des Tyr$_{97}$ 1,06 nm; C des terminalen $CO_2$ zu C des $CO_2$ Glu$_{98}$ 0,50 nm; C des $CO_2$ Glu$_{98}$ zu C-OH des Tyr$_{97}$ 1,13 nm; C des $CO_2$ Glu$_{98}$ zu C-OH des Tyr$_{99}$ 0.85 nm. Anstatt von Tyr$_{99}$, sind Leu oder Try gute Alternativen, aber die phenolische OH-Gruppe des Tyrosins kann für die Entwicklung von Mimetika wertvoll sein.

Die Erfindung wird durch das Beispiel erläutert.

Die in dem Beispiel verwendeten Peptide wurden in üblicher Weise synthetisiert. Um die Tabellen übersichtlich zu gestalten, wurde in diesen der übliche einbuchstabige Code für Aminosäuren verwendet. Sonderformen von Aminosäuren werden in den Tabellen entsprechend angegeben.

**Beispiel**

Es wurden die in den Tabellen 1 bis 5 angegebenen Peptide untersucht. Dabei wurden die IC$_{50}$-Werte zur Hemmung der HIV-1 -Protease und HIV-2-Protease in einem üblichen Test (L.H Phylip et al., Biochemical and Biophysical Research Communications, 117, 439-444 (1990)) bestimmt. Der IC$_{50}$-Wert gibt die Konzentration des Peptids an, bei der eine 50%ige Hemmung der Proteaseaktivität zu beobachten ist. Die Ergebnisse sind ebenfalls in den Tabellen 1 bis 5 gezeigt.

Aus diesen geht hervor, daß die erfindungsgemäßen Peptide eine hohe Hemmaktivität für retrovirale Proteasen haben.

Tabelle 1. Inhibition der HIV Proteasen durch C-terminale 'Interface' Peptide

| Peptid | $IC_{50}$ ($\mu$M) (HIV-1) | $IC_{50}$ ($\mu$M) (HIV-2) |
|---|---|---|
| *H*–S–F–N–L–*OH* | 900 | – |
| *H*–S–Y–N–L–*OH* | 440 | – |
| *H*–I–S–Y–E–Y–*OH* | 37 | 20 |
| *H*–I–S–Y–E–**W**–*OH* | 10 | 70 |
| *H*–R–S–Y–E–Y–*OH* | 120 | – |
| *Ac*–H–M–S–F–N–L–*OH* | 150 | 50 |
| *H*–T–V–S–F–N–F–*OH* | 28 | 50 |
| *Ac*–T–V–S–F–N–F–*OH* | 140[*] | – |
| *Ibu*–T–V–S–F–N–F–*OH*[1] | <62 l.s. | – |
| *Pam*–T–V–S–F–N–F–*OH*[2] | 15 l.s. | 20 |
| *Ac*–R–T–V–S–F–N–F–*OH* | 180 | – |
| *Ac*–R–V–S–F–N–F–*OH* | 20 | 72 |
| *H*–D–R–V–S–F–N–F–*OH* | 200 | – |
| *Ac*–T–V–S–Y–N–L–*OH* | 100 | – |
| *Ac*–T–V–S–Y–E–L–N–*OH* | 100 | – |
| *Ac*–T–V–S–Y–E–L–*OH* | 8 | 1.8 |
| *Ibu*–T–V–S–Y–E–L–*NH*$_2$[1] | 175 | – |
| *H*–T–V–S–Y–E–L–*OH* | 12 | 4 |
| *H*–T–V–S–Y–E–**W**–*OH* | 18 | 85 |
| *H*–T–V–S–R–E–L–*OH* | 150 | – |
| *H*–T–R–S–Y–E–L–*OH* | <40 | 5 |
| *H*–T–I–S–Y–E–L–*OH* | 3 | 1.5 |
| *H*–R–I–S–Y–E–Y–*OH* | <20 | – |
| *Ac*–N–R–R–S–Y–E–L–N–*OH* | 300 | – |
| *Ibu*–N–R–R–S–Y–N–F–*OH*[1] | 190 | – |
| *Ac*–R–R–S–Y–N–L–*OH* | 260 | – |
| *Ac*–I–G–M–T–L–N–(C–)–(C–)–*NH*$_2$[**] | 70%[+] | – |
| *Ac*–T–L–N–(C–)–(C–)–L–*OH*[**] | 44%[+] | – |

– = nicht bestimmt; l.s. = geringe Löslichkeit ; [1] *Ibu*- = Isobutyroyl-; [2] *Pam*- = Palmitoyl-; [*] Ki = 6.1μM; [**] cyclisches Peptid mit -SS-Bindung; [+] bei 800 μg/ml.

Tabelle 2

| Inhibition der HIV-1 Protease durch bifunktionelle Peptide | |
|---|---|
| Peptide | IC$_{50}$ ($\mu$M) |
| H-P-Q-I-T-L-S-D-R-L-F-G-G-R-S-V-E-F-N-F-OH | 30 |
| H-P-Q-I-T-L-S-D-R-L-F-G-G-D-R-V-S-F-N-F-OH | 10 |
| H-P-Q-I-T-L-E-N-L-T-G-G-V-S-F-N-F-OH | 10 |
| H-P-E-I-T-L-S-D-R-L-G-G-G-D-R-V-S-F-N-F-NH$_2$ | 100 |
| H-P-Q-I-T-L-S-D-R-L-G-G-G-G-D-R-V-S-F-N-F-NH$_2$ | 36%$^+$ |
| H-L-E-I-T-L-S-D-R-L-G-G-G-D-R-V-S-F-N-F-NH$_2$ | 15 |
| H-L-E-I-T-L-G-E-R-L-G-G-G-G-D-R-V-S-F-N-F-NH$_2$ | 5 |
| H-L-E-I-T-L-G-E-R-G-G-G-G-G-D-R-I-S-Y-E-L-OH* | 1 |
| H-L-E-I-T-L-W-E-R-X-X-I-S-Y-E-L-OH* | 0.5 |
| H-E-I-T-L-E-N-X-X-X-I-S-Y-E-L-OH* | 0.5 |
| H-L-E-I-T-L-E-N-$\beta$-$\beta$-$\beta$-$\beta$-$\beta$-I-S-Y-E-L-OH* | 1 |
| Mögliche Bindungssegmente sind unterstrichen; Tests mit 5% DMSO, *kein DMSO; $^+$bei 50 $\mu$M, nur 1 Bestimmung; X = 6-aminohexanoic acid; $\beta$ = $\beta$-Alanin. | |

Tabelle 3. Inhibition der HIV-1 Protease durch N-terminale 'Interface' Peptide

| Peptide | IC$_{50}$ ($\mu$M) | % Inhibition (800 $\mu$g/ml) |
|---|---|---|
| H-P-Q-I-T-L-W-Q-R-P-L-V-T-I-K-I-OH | 830 | – |
| Ac-P-Q-I-T-L-W-Q-R-P-NH$_2$ | – | 7 |
| Ac-P-Q-I-T-L-W-Q-R-NH$_2$ | – | 28 |
| Ac-P-Q-I-T-L-NH$_2$ | – | 10 |
| H-L-Q-I-T-L-W-OH | 37 | – |
| H-L-Q-V-T-F-(DS)-E-R-(DS)-F-NH$_2$ | 120 | – |
| H-L-Q-V-T-F-(DS)-R-(DS)-F-NH$_2$ | 120 | – |
| H-L-Q-V-T-F-(DS)-E-R-NH$_2$ | 120 | – |
| H-L-Q-C(acm)-T-L-G-E-R-C(acm)-A-OH$^\#$ | 230 | – |
| H-L-Q-C(S-)-T-L-G-E-R-C(S-)-A-OH$^{\#\#}$ | 350 | – |

" - " = nicht bestimmt; $^\#$ acm = acetamidomethyl Gruppe ; $^{\#\#}$ -S-S-Bindung , cyclisches Peptid; DS = D-Ser;

Tabelle 4: Inhibition der HIV-Proteasen durch Interface-Peptide

| Peptide | $IC_{50}$ (HIV-1) ($\mu M$) | $IC_{50}$ (HIV-2) ($\mu M$) |
|---|---|---|
| S-Y-N-L | 440 | - |
| S-Y-E-L | 80 | - |
| Ac-S-Y-E-L | 55 | - |
| S-Y-E-Y | 75 | - |
| S-Y-E-W | 75 | - |
| (w)-Y-E-W | 25 | - |
| (w)-Y-E-L | 27 | - |
| (w)-Y-D-L | 12 | - |
| Y-E-L | 85 | - |
| Bio-Y-E-L | 6 | - |
| Y-S-Y-E-L | 5.5 | - |
| I-S-Y-E-L | 4.5 | - |
| Ac-I-S-Y-E-L | 3.6 | - |
| Ac-I-S-Y-N-L | 70 | - |
| I-S-Y-E-Y | 37 | 20 |
| I-S-Y-E-W | 10 | 70 |
| I-T-Y-E-W | 20 | - |
| R-S-Y-E-Y | 120 | - |
| R-I-S-Y-E-Y | 20 | - |
| V-S-Y-C(SEt)-Y | 120 | - |
| Ac-H-M-S-F-N-L | 150 | 50 |
| Ibu-T-V-S-F-N-F | <62 | - |
| Pam-T-V-S-F-N-F | 15 | 20 |
| Ac-R-V-S-F-N-F | 20 | 70 |
| Ac-R-R-S-Y-N-L | 260 | - |
| Ac-T-V-S-Y-N-L | 100 | - |
| Ac-T-V-S-Y-E-L | 8 | 2 |
| Bio-T-V-S-Y-E-L | 5 | - |
| Cap-T-V-S-Y-E-L | 3 | - |
| Ibu-T-V-S-Y-E-L-$NH_2$ | 175 | - |
| Ibu-T-V-S-Y-E-L | 6 | - |
| Pam-T-V-S-Y-E-L | 0.5 | 1.3 |
| T-V-S-Y-E-L | 12 | 4 |
| T-I-S-Y-E-L | 6 | 1.5 |
| T-V-S-Y-E-W | 7 | 85 |
| T-R-S-Y-E-L | 23 | 5 |
| Y-I-S-Y-E-L | 6 | - |
| I-I-S-Y-E-L | 8 | - |
| Ac-T-V-S-Y-E-L-N | 100 | - |

- = nicht bestimmt; Ibu = Isobutyroyl-; Pam = Palmitoyl-;
Bio = Biotinyl-; Cap = Caproyl-; (w) = D-Trp

Tabelle 5

| Inhibition der HIV-1 Protease durch N-terminale 'Interface'Peptide. | |
|---|---|
| Peptides | $IC_{50}{}^*$ ($\mu$M) |
| P-Q-I-T-L-W-Q-R-P-L-V-T-I-K-I | 830 |
| L-Q-I-T-L-W | 37 |
| L-Orn-L-K-K-(w)-*NH₂* | 700 |
| L-Q-V-T-F-(s)-E-R-(s)-F-NH₂ | 120 |
| L-Q-V-T-F-(s)-R-(s)-F-*NH₂* | 120 |
| L-Q-V-T-F-(s)-E-R-*NH₂* | 120 |
| L-Q-C(acm)-T-L-G-E-R-C(acm)-A[#] | 230 |
| L-Q-C(S-)-T-L-G-E-R-C(S-)-A[##] | 350 |
| L-Q-I-T-C(SEt)-W | 12 |
| L-Q-I-T-F-(y) | 130 |

[#]acm = Acetamidomethyl-Gruppe;
[##] cyclisches Peptid mit einer S-S-Bindung zwischen den Halb-Cystin-Resten C(S-); (s), (w), (y) = D-Ser, D-Trp-, D-Tyr.

Tabelle 6: Consensus Struktur von bevorzugten, potenten C-terminalen 'Interface' Inhibitoren

Eigenschaften der Aminosäureseitenketten:

Position 95: mittel oder groß, hydrophob; auch D-Aminosäuren: hydrophob oder mit positiver Ladung

Position 96: mittel, hydrophil; große, hydrophobe D-Aminosäure

Position 97: groß, hydrophob, Wasserstoff-gebunden; auch D-Form

Position 98: negativ geladen nur L-Aminosäuren

Position 99: groß, hydrophob; aliphatische D-Aminosäuren

bevorzugte Beispiele:

| Position | 94 | 95 | 96 | 97 | 98 | 99 |
|---|---|---|---|---|---|---|
| | (Thr) | Val | Ser | Tyr | Glu | Leu-OH |
| | | Ile | Thr | | Asp | Trp-OH |
| | | Trp | Asn | | | Tyr-OH |
| mögliche D-Aminosäuren | | orn | trp | tyr | | leu-OH |
| | | ile | phe | | | phe-OH |

Der C-Terminus 99 sollt frei sein (negativ geladen); N-terminale Blocks sind in den Positionen 94 bis 97 (hexa- bis Tripeptid) möglich. Kurze Peptide der Reste 95 (96) bis 99 zeigen eine bessere Korrelation zu den Docking-Daten als einige der langen Peptide.

**Patentansprüche**

1. Peptid, dadurch gekennzeichnet, daß das Peptid eine Aminosäuresequenz der allgemeinen Formel (1) umfaßt

   $Y-A^2-A^3-A^4-X$     (1),

   in der
   | | |
   |---|---|
   | Y | Wasserstoff, eine Aminosäure oder ein weiteres Peptid ist, |
   | $A^2$ | Arg, Tyr oder Phe ist, |
   | $A^3$ | Glu, Gln, Asn oder Asp ist, |
   | $A^4$ | Leu, Phe, Met, Tyr, Trp, Ile oder Nle ist und |
   | X | OH, $NH_2$, eine Aminosäure oder ein weiteres Peptid ist. |

2. Peptid nach Anspruch 1, dadurch gekennzeichnet, daß Y eine Aminosäure ist, ausgewählt aus Ser, Thr, HomoSer, Asn, Glu, Typ, Phe, Leu oder Ile.

3. Peptid nach Anspruch 1, dadurch gekennzeichnet, daß Y ein weiteres Peptid der allgemeinen Formel (2) ist

   $R^3-A^1$     (2),

   in der
   | | |
   |---|---|
   | $A^1$ | Ser, Thr, HomoSer, Asn, Glu, Typ, Phe, Leu oder Ile ist und |
   | $R^3$ | Ile, Leu, Met, Phe, Tyr, Val, Orn, Lys oder Arg ist. |

4. Peptid nach Anspruch 1, dadurch gekennzeichnet, daß Y ein weiteres Peptid der allgemeinen Formel (3) ist

   $R^2-R^3-A^1$     (3),

   in der
   | | |
   |---|---|
   | $R^2$ | Lys, Thr, His, Met, Ile oder Arg ist und |
   | $R^3$ und $A^1$ | wie in Anspruch 3 definiert sind. |

5. Peptid nach Anspruch 1, dadurch gekennzeichnet, daß Y ein weiteres Peptid der allgemeinen Formel (4) ist,

   $R^1-R^2-R^3-A^1$     (4),

   in der
   | | |
   |---|---|
   | $R^1$ | Arg, Asp oder Asn ist und |
   | $R^2$ | wie in Anspruch 4 und |
   | $R^3$ und $A^1$ | wie in Anspruch 3 definiert sind. |

6. Peptid nach einem der Ansprüche 1 bis 5, dadurch gekennzeichent, daß X eine Aminosäure ist, ausgewählt aus Glu, Gln, Asp oder Asn.

7. Peptid nach einem der Ansprüche 1 bis 5, dadurch gekennzeichnet, daß X ein weiteres Peptid der allgemeinen Formel ist

   $R^4-R^5$     (5),

   in der
   | | |
   |---|---|
   | $R^4$ | Glu, Gln, Asp oder Asn ist und |
   | $R^5$ | Met, Leu, Phe, Tyr, Leu, oder Met ist. |

8. Peptid nach einem der Ansprüche 1 bis 7, dadurch gekennzeichent, daß an das N-terminale Ende des Peptids über einen Linker ein Peptidfragment oder modifiziertes Peptidfragment einer HIV-Protease gebunden ist.

9. Peptid nach einem der Ansprüche 1 bis 8, dadurch gekennzeichnet, daß an das Peptid oder an das Peptidfragment bzw. modifizierte Peptidfragment einer HIV-Protease eine Amidin-Gruppe, eine Acyl-Gruppe, ein aliphatischer Rest mit 1 bis 20 Kohlenstoffatomen, ein aromatischer Rest mit 1 bis 20 Kohlenstoffatomen, ein Rest mit 1 bis 20 Kohlenstoffatomen aus einem aromatischen und aliphatischen Teil, ein Vitamin oder -derivat, ein Steroid, ein Hormon, ein Antigen, ein an einen Rezeptor bindendes Peptid, ein Toxin, ein Inhibitor, ein Cytokin und/oder ein Borkomplex für Neutronentherapie gebunden ist.

10. Peptid nach Anspruch 9, dadurch gekennzeichnet, daß die Acyl-Gruppe von Essigsäure, Isobuttersäure oder Palmitinsäure abgeleitet ist.

11. Peptid nach einem der Ansprüche 1 bis 10, dadurch gekennzeichnet, daß mindestens eine Aminosäure des Peptids chemisch modifiziert ist.

12. Verfahren zur Herstellung eines Peptids nach Anspruch 1, wobei die Aminosäuren in an sich bekannter Weise miteinander verbunden werden.

13. Verwendung einer Verbindung nach einem der Ansprüche 1 bis 11 als Arzneimittel.

Figur 1:    Stereoplot des gebundenen Interfacepeptides SYEY

Europäisches
Patentamt

# EUROPÄISCHER TEILRECHERCHENBERICHT
Nummer der Anmeldung

der nach Regel 45 des Europäischen Patentübereinkommens für das weitere Verfahren als
europäischer Recherchenbericht gilt

EP 95 10 5382

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | BIOCHEMICAL AND BIOPHYSICAL RESEARCH COMMUNICATIONS., Bd.194, Nr.2, 30. Juli 1993, DULUTH, MINNESOTA US Seiten 595 - 600 H J SCHRAMM ET AL 'The inhibition of HIV-1 protease by interface peptides' * das ganze Dokument * --- | 1-13 | C07K5/10 C07K5/08 C07K14/16 A61K38/04 |
| X | BIOCHEMICAL AND BIOPHYSICAL RESEARCH COMMUNICATIONS., Bd.179, Nr.2, 1991, DULUTH, MINNESOTA US Seiten 847 - 851 H J SCHRAMM ET AL. 'HIV-1 reproduction is inhibited by peptides derived from the N- and C-termini of HIV-1 protease' * das ganze Dokument * --- -/-- | 1-13 | |

RECHERCHIERTE
SACHGEBIETE (Int.Cl.6)

C07K
A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich
ist, auf der Grundlage einiger Patentansprüche sinnvolle Ermittlungen über den Stand der
Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 17. Juli 1995 | Masturzo, P |

| | **EINSCHLÄGIGE DOKUMENTE** | | **KLASSIFIKATION DER ANMELDUNG (Int.Cl.6)** |
|---|---|---|---|
| **Kategorie** | **Kennzeichnung des Dokuments mit Angabe, soweit erforderlich der maßgeblichen Teile** | **Betrifft Anspruch** | |
| X | BIOCHEMICAL AND BIOPHYSICAL RESEARCH COMMUNICATIONS., Bd.184, Nr.2, 30. April 1992, DULUTH, MINNESOTA US Seiten 980 - 985 H J SCHRAMM ET AL. 'Inhibition of HIV-1 protease by short peptides derived from the terminal segments of the protease' * das ganze Dokument * --- | 1-13 | |
| X | FEBS LETTERS., Bd.281, Nr.1,2, 9. April 1991, AMSTERDAM NL Seiten 77 - 80 J TÖZSÉR ET AL. 'Comparison of HIV-1 and HIV-2 proteinases using oligopeptide substrates representing cleavage sites in Gag and Gag-Pol polyproteins' * das ganze Dokument * --- | 1-13 | **RECHERCHIERTE SACHGEBIETE** (Int.Cl.6) |
| X | FEBS LETTERS., Bd.247, Nr.1, September 1989, AMSTERDAM NL Seiten 118 - 122 I V PECHIK ET AL. 'Possible rôle of some groups in the structure and function of HIV-1 protease as revealed by molecular modeling studies' * das ganze Dokument * --- | 1-13 | |
| X | JOURNAL OF BIOLOGICAL CHEMISTRY., Bd.266, Nr.24, 25. August 1991, BALTIMORE US Seiten 15591 - 15594 ZHONG-YIN ZHANG ET AL. 'Dissociative inhibition of dimeric enzymes' * das ganze Dokument * --- -/-- | 1-13 | |

EPO FORM 1503 03.82 (P04C12)

| EINSCHLÄGIGE DOKUMENTE | | | KLASSIFIKATION DER ANMELDUNG (Int.Cl.6) |
| --- | --- | --- | --- |
| Kategorie | Kennzeichnung des Dokuments mit Angabe, soweit erforderlich der maßgeblichen Teile | Betrifft Anspruch | |
| X | BIOLOGICAL CHEMISTRY HOPPE-SEYLER, Bd.374, Nr.9, September 1993 Seite 712 H J SCHRAMM ET AL. 'The inhibition of HIV-1 protease by interface peptides' *Zusammenfassung II20* --- | 1-13 | |
| X | PROTEIN SCIENCE, Bd.1, 1992, CAMBRIDGE, MASS. Seiten 1244 - 1253 L M BABÉ ET AL. 'Synthetic "interface" peptieds aalter dimeric assembly of the HIV-1 and HIV-2 proteases' * das ganze Dokument * --- | 1-13 | |
| X | WO-A-90 09191 (W SCHRAMM ET AL.) 23. August 1990 * das ganze Dokument * ----- | 1-13 | **RECHERCHIERTE SACHGEBIETE** (Int.Cl.6) |

EP 95 10 5382

-C-

Bemerkung: Obwohl Anspruch 13
sich auf ein Verfahren zur Behandlung des
menschlichen/tierischen Körpers
(Diagnostizierverfahren, das am menschlichen/
tierischen Körper vorgenommen wird,)
bezieht (Art. 52(4)EPU), wurde die
Recherche durchgeführt und gründete sich auf
die angeführten Wirkungen der Verbindung/
Zusammensetzung.